# NEUE EUROPÄISCHE PATENTSCHRIFT

(11) **EP 0 114 611 B2**
(45) Veröffentlichungstag und Bekanntmachung des Hinweises auf die Entscheidung über den Einspruch: **02.11.1994**
(45) Hinweis auf die Patenterteilung: 03.09.1986
(21) Anmeldenummer: 84100209.0
(22) Anmeldetag: 11.01.1984
(51) Int. Cl.: C07C 45/50, C07C 47/02

(54) **Verfahren zur kontinuierlichen Hydroformylierung olefinisch ungesättigter Verbindungen**
Process for continuous hydroformylation of olefinic unsaturated compounds
Procédé d'hydroformylation en continu de composés à insaturation oléfinique

(30) Priorität: 19.01.1983 DE 3301591
(43) Veröffentlichungstag der Anmeldung: 01.08.1984
(73) Patentinhaber: BASF Aktiengesellschaft, 67063 Ludwigshafen (DE)
(72) Erfinder: Kummer, Rudolf, Dr., D-6710 Frankenthal (DE); Richter, Wolfgang, Dr., D-6700 Ludwigshafen (DE); Schwirten, Kurt, Dr., D-6710 Frankenthal (DE); Stops, Peter, D-6701 Altrip (DE)

(56) Entgegenhaltungen:
- EP-A- 0 007 768
- EP-A- 0 016 285
- EP-A- 0 016 286
- EP-A- 0 062 282
- EP-B- 0 016 286
- DE-A- 2 715 685
- GB-A- 826 763
- GB-A- 1 002 428
- GB-A-13 120 76
- GB-A-98 894 1
- US-A- 3 418 351
- US-A- 3 527 809
- US-A- 4 166 773
- US-A- 4 242 283
- US-A-35 768 81
- US-A-36 814 65
- US-A-41 488 30
- US-A-42 474 86
- SRJ-Report n 21B (1978), S. 139-143, Abt. 8.1
- SRJ-Report n 21B (1978), S. 67-75, Abt. 6.1
- T 516/89
- "Syngas reactions part VIII : The preparation of glycol monoalkyl Ehters" by Johm F. Knipton, Journal of Molecular Catalysis, 30 (1985), 281-297
- "Reaction Kinetics of the Aldol Condensation of mixed C7 Aldehydes" by F.J. Doering and G.F. Schaefer, Journal of Molecular Catalysis, 41 (1987) 313-328
- J. Falbe, New Synthesis with carbon monoxide (1980), S. 173
- Chemical Engineering. 5.12.77, pages 110-117

## Beschreibung

Die vorhegende Erfindung betrifft ein verbessertes Verfahren zur kontinuierlichen Hydroformylierung von olefinisch ungesättigten Verbindungen bei 2 bis 30 bar und 80 bis 130 _{°} C mit Hilfe von Rhodium-Komplexverbindungen als Katalysatoren, welche schwerflüchtige Verbindungen der allgemeinen Formel I

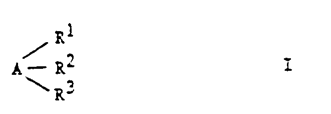

in der A für Phosphor, Arsen. Antimon oder Wismut steht und in der R¹⁻R³ organische Reste bedeuten, als Liganden enthalten.

Dieses Verfahren ist, sieht man von der erfindungsgemäßen Verbesserung ab. allgemein bekannt. z.B. aus der DE-A 11 86 455 und der DE-A 1793069.

Werterhin ist es allgemein bekannt, die Verfahrensprodukte - vornehmlich Aldehyde sowie daneben auch die entsprechenden Alkohole - entweder dadurch zu gewinnen, daß man das flüssige, von gasförmigen Bestandteilen befreite Reaktionsgemisch destillativ aufarbeitet oder daß man sie nach der sogenannten Kreisgasfahrweise zusammen mit den gasförmigen Reaktionspartnern gasförmig aus dem Hydroformylierungsreaktor austrägt und aus dem Gasstrom abtrennt, wonach die Gase größtenteils wieder in den Reaktor zurückgeführt werden, wie es beispielsweise in der DE-A 27 15 685 beschrieben ist.

Die erstgenannte Methode hat den grundsätzlichen Nachteil, daß der Katalysator, welcher in dem flüssigen Reaktionsgemisch enthalten ist, unter den Destillationsbedingungen, hauptsächlich wegen der Abwesenheit der CO/H₂-Atmosphäre, in deren Gegenwart die Hydroformylierung stattfindet, geschädigt oder zumindest desaktiviert wird.

Bei der Kreisgasfahrweise, bei welcher der Katalysator im Hydroformylierungsreaktor verbleibt, kann der geschilderte Nachteil zwar nicht auftreten, jedoch ist diese Methode nur dann praktikabel, wenn die Verfahrensprodukte einen hinreichend hohen Partialdruck haben, um in genügender Menge mit dem Kreisgas ausgetragen werden zu können. Ist der Partialdruck hingegen sehr gering, würde man zum Austrag unverhältnismäßig große Gasmengen benötigen, wodurch das Hydroformylierungsverfahren unwirtschaftlich werden würde.

Die Möglichkeit, die Kreisgasfahrweise effektiver zu gestalten, indem man die Hydroformylierungstemperatur und damit den Partialdruck der Verfahrensprodukte erhöht, ist nicht sinnvoll, weil man sich auf diese Weise vom Temperaturoptimum der Hydroformylierung entfernen und schlechtere Verfahrensergebnisse erzielen würde, sei es, daß unerwünschte Hydrierungen vom Olefin zum Paraffin oder vom Aldehyd zum Alkohol stattfinden, sei es wegen der mit höheren Temperaturen zunehmenden Isomerisierung der Olefine oder sei es wegen der Verminderung des Anteils der meist bevorzugten linearen Aldehyde.

Der Erfindung lag daher die Aufgabe zugrunde, den genannten Nachteilen abzuhelfen und die Gewinnung der Verfahrensprodukte aus den Hydroformylierungsgemischen wirksamer zu gestalten.

Demgemäß wurde ein verbessertes Verfahren zur kontinuierlichen Hydroformylierung von a-Olefinen mit 5 bis 12 C- Atomen bei 2 bis 30 bar und 80 bis 130 _{°} C mit Hilfe von Rhodium-Komplexverbindungen als Katalysatoren, die schwer flüchtige Verbindungen der allgemeinen Formel I

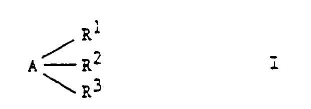

In der A für Phosphor, Arsen, Antimon oder Wismut steht und in der R¹ bis R³ organische Reste bedeuten als Liganden enthalten, mittels Kreisgasfahrweise, gefunden, welches dadurch gekennzeichnet ist, daß man das aus flüssigen und gasförmigen Bestandteilen bestehende Hydroformylierungsgemisch dem Reaktor entnimmt, in einer Entgasungskolonne kurzzeitig höheren Temperaturen und/oder niedrigeren Drücken aussetzt und dabei gleichzeitig eine Trennung des Gemisches in eine Gas- und eine Flüssigphase vornimmt, die Gasphase in einem Produktabscheider in Produkt und Kreisgas trennt und das Kreisgas sowie die Flüssigphase der Entgasungskolonne wieder in den Reaktor zurückführt.

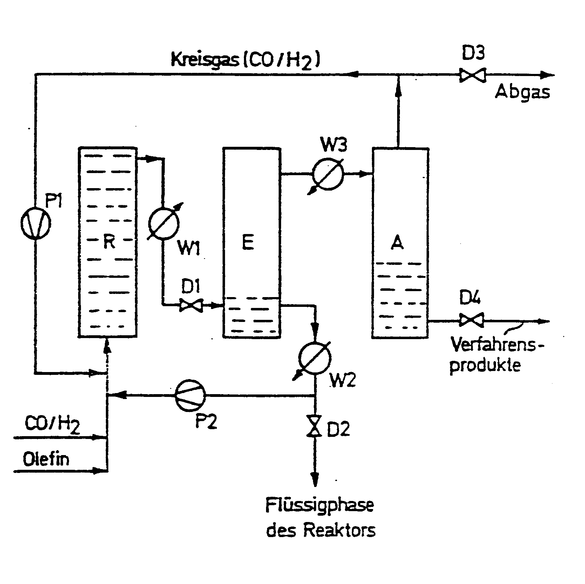

Das Verfahren sei anhand der Zeichnung erläutert:

Das den Hydroformylierungsreaktor R verlassende, teils flüssige, teils gasförmige Reaktionsgemisch wird zweckmäßigerweise in einem Wärmeaustauscher W1 auf die höhere Temperatur gebracht, mit welcher die Entgasungskolonne E erfindungsgemäß betrieben wird.

Man kann die Entgasung in E unter dem gleichen Druck wie in R vornehmen, jedoch empfiehlt es sich, den Druck über das Drosselventil D1 abzusenken.

In E findet sodann die Trennung des Reaktionsgemisches in eine Gas- und eine Flüssigphase statt Die hierfür benötigte Verweilzeit von etwa 2 bis 10 min ist ungleich kürzer als die in R für die Hydroformylierung erforderliche Verweilzeit, die ungefähr 4 bis 8 Stunden beträgt, so daß trotz der höheren Temperatur praktisch keine unerwünschten Neben- und Nachreaktionen zu beobachten sind, obwohl hier das Reaktionsgemisch weiterhin unter Hydroformylierungsbedingungen steht.

Die Flüssigphase, welche die Entgasungskolonne verläßt, kann, sofern erforderlich, im Wärmetauscher W2 abgekühlt werden. In diesem Falle ist es wärmetechisch zweckmäßig. W1 und W2 sowie eventuell auch W3 in einer gemeinsamen Vorrichtung zusammenzufassen (in der Zeichnung nicht dargestellt).

Die Temperatur in der Entgasungskolonne E soll in der Regel mindestens 5 _{°} C höher sein als die Hydroformylierungstemperatur. In der Praxis liegt diese Temperaturdifferenz jedoch meist zwischen 10 und 50 _{°} C.

Im Hinblick auf den gasförmigen Austrag der Verfahrensprodukte von der Entgasungskolonne E ist auch der Druck von Bedeutung und könnte gleich oder auch geringer sein als der Hydroformylierungsdruck, weil bei vermindertem Gesamtdruck entsprechend dem durch die Temperstur bestimmten Partialdruck der Volumenanteil der Hydroformylierungsprodukte im Gasstrom zunimmt. Deshalb empfiehlt es sich im allgemeinen, mit der Temparaturerhöhung auch den Druck zu senken. Diese Druckdifferenz beträgt vorzugsweise 2 bis 20 bar, und zwar selbstverständlich mit der Maßgabe, daß sie nicht größer als (p-1) sein darf wobei p der Hydroformylierungsdruck ist.

Da die Einstellung der Partialdruck-Gleichgewichte in E sehr rasch verläuft, sind dort nur Verweilzeiten von 1 bis 30, in der Regel von 2 bis 10 min erforderlich.

Da sich die Flüssigphase von R im Laufe der Zeit mit hochsiedenden Reaktionsprodukten anreichert, ist es erforderlich, ab und zu einen Teil dieser Flüssigphase über das Drosselventil D2 auszuschleusen Das gleiche gilt für die Abgas-Ausschleusung über D3. Beide Maßnahmen und Vorrichtungen sind jedoch nicht erfindungswesentlich und wurden daher nur der Vollständigkeit halber erwähnt.

Über die Pumpe P2, welche dir unvermeidlichen sowie auch gezielten Druckverluste ausgleicht gelangt die Flüssigphase zusammen mit frischem Synthesegas (CO/H₂), frischem Olefin und dem Kreisgas wieder in den Reaktor zurück.

Die Gasphase von E wird im Abscheider A oder zweckmäßigerweise in einem vorgeschaltenen Wärmetauscher W3 wie üblich soweit abgekühlt, daß sich die Verfahrensprodukte vornehmlich die Aldehyde und daneben auch die Alkohole sowie nicht umgesetztes Olefin und gebildetes Paraffin flüssig abscheiden, wonach sie über das Entspannungsventil D4 aus dem System entnommen und wie üblich weiterbehanden werden. Die Gasphase, das hauptsächlich aus CO, H₂, N₂ und gegebenenfalls auch aus geringen Mengen Olefin und dem entsprechenden Paraffin bestenende sogenannte Kreisgas, wird nach Abtrennung eines Abgas-Teilstroms über D3 ebenfalls wie üblich über den Kompressor P1 wieder in den Hydroformylierungsreaktor zurückgeführt.

Das erfindungsgemäße Verfahren besteht somit in einer sinnvollen Entkopplung der Bedingungen für eine optimale Hydroformylierung und einen optimalen gasförmigen Produktaustrag nach der Kreisgasfahrweise, im übrigen ist das Verfahren innerhalb der definitionsgemäßen allgemeinen Rahmenbedingungen für die Hydroformylierung von der Art der Hydroformylierung unabhängig, so daß hier einige grundsätzliche Erläuterungen genügen.

Als olefinisch ungesättigte Verbindungen, die hier gelegentlich auch kurz als "Olefine" bezeichnet werden, kommen a-Olefine mit 5 bis zu. 12 C-Atomen in Betracht, daneben aber auch beispielsweise andere a-olefinish ungesättigte Verbindungen wie Allylalkohol, Allylacetat, Acrylsäureester, Styrol und Acroleinacetale.

Olefinisch ungesättigte Verbindungen mit innenständiger Doppelbindung werden unter den angegebenen Reaktionsbedingungen in der Regel nicht oder nur in geringem Umfang hydroformyliert. Für Ausnahmefähe wäre des erfindungsgemäße Verfahren aber selbstverständlich gleichwohl geeignet.

Obwohl sich die Hydroformylierung der niederen Olefine wie Ethylen, Propylen und But-1-en mit Hilfe des erfindungsgemäßen Verfahrens ebenfalls effektiver gestalten läßt, so hat es doch die größere Bedeutung im Falle von a-Olefinen mit 5 bis 12 C-Atomen, da der Partialdruck der entstehenden Aldehyde bei der Hydroformylierungstemperatur so gering ist, daß man das Kreisgas unverhältnismäßig oft, also mit erheblichem Energieaufwand, zirkulieren lassen müßte.

Charakteristisch für die mit Rhodium katalysierte Hydroformylierung ist die Mitverwendung der komplexbildenden Liganden I, die in der Regel in 3 bis 500-fachem molaren Übetschuß über das Rhodium eingesetzt werden. Meist geht man nicht von einem fertigen Rh-Komplex dieser Art aus, weil sich die Komplexe unter den Hydroformylierungsbedingungen aus Rh-Salzen. z.B. dem Acetat, und den Liganden I in situ bilden. Unter den zahlreichen bekannt gewordenen Liganden - vgl. z.B. die eingangs zitierte Vorliteraturhaben die Phosphorverbindungen wie Trialkyl- und Triarylphosphine sowie Trialkyl- und Triarylphosphite praktisch alleinige wirtschaftliche Bedeutung, und unter diesen Liganden wird wiederum das Triphnenylphosphin bevorzugt. Im Einzelfall richtet sich die Wahl des Liganden nach den speziellen Hydroformylierungsaufgaben, die im Rahmen der vorliegenden Erfindung jedoch nicht erfindungskritisch sind.

Aus praktischen Gründen ist hier lediglich zu beachten, daß die Liganden I hinreichend schwerflüchtig sein sollen, daß sie höchstens in Spuren in die Gasphase der Entgasungskolonne E gelangen, da sie andernfalls den Rohaldehyd verunreinigen und dessen Aufarbeitung erschweren würden.

Die Rhodium-Konzentration liegt meist im üblichen Bereich, also etwa zwischen 50 und 500 ppm des Reaktionsgemisches.

Das Molverhältnis von CO zu H₂ kann je nach der Hydroformylierungsaufgabe zwischen etwa 10: 90 bis 90: 10 liegen. Im allgemeinen liegt es, besonders wenh Aldehyde als Verfahrensprodukte erwünscht sind, zwischen 45: 55 und 55: 45.

Das erfindungsgemäße Verfahren gestattet es, etwa die 5 bis 20-fache Menge der Verfahrensprodukte mit der gleichen Kreisgasmenge zu gewinnen als nach der Kreisgasfahrweise des Standes der Technik. Selbstverständlich ist-es auch möglich, die Kreisgasmenge, die beim herkömmlichen Verfahren in der Rege, das 100- bis 200-fache der Frischgasmenge beträgt, entsprechend zu reduzieren. Bei erfindungsgemäßen Verfahren richtet man es zweckmäßigerweise so ein, daß die Kreisgasmenge das 10-bis 30-fache der Frischgasmenge beträgt.

Besondere Bedeutung hat das erfindungsgemäße Verfahren für die Herstellung von C₆-C₁₃-Alkanalen aus den entsprechenden a-Olefinen. Diese Aldehyde werden vornehmlich zu den entsprechenden Alkoholen reduziert, die als Komponenten von Kunststoff-Weichmachern des Estertyps Verwendung finden Ferner werden sie zu den entsprechenden Carbonsäuren oxidiert, die als Bestandteile von Schmierstoffen technische Bedeutung haben.

### Beispiel

### > droformylierung von Oct-1-en

Ein Hydroformylierungs-Versuchsreaktor R von 40 I Inhalt wurde stündlich mit 2.2 kg Oct-1-en, 0.94 Nm³ eines Gemisches aus CO und H₂ im Volumenverhältnis von 48: 52, mit 24 Nm³ eines Kretsgases, das im wesentlichen aus 80 Vol.% H₂ 15 Vol.% CO und 5 Vol.% N₂ bestand, sowie mit 4.0 kg rückgeführter Flüssigkeit beschickt.

Das Volumenverhältnis von Kreisgas zu Frischgas betrug somit rd. 26.1.

Die Hydroformylierungstemperatur betrug 105°C und der Druck 14 bar.

Die Konzentration der Katalysatorbestandteile betrug 100 ppm Rhodium (als Rh-Acetat eingesetzt) und 4.7 Gew.% Triphenylphosphin (Molverhältnis Rh: Phosphin = 1: 185).

Das den Reaktor verlassende Reaktionsgemisch, welches neben dem Kreisgas etwa 6.7 kg flüssige Bestandteile enthielt, wurde im Wärmetauscher W1 auf 120 _{°} C aufgeheizt, auf 3 bar entspannt und in die Entgasungskolonne E gegeben wobei sich das Kreisgas mit den Verfahrensprodukten belud

Die katalysatorhaltige Flüssigphase von E wurde im Wärmetauscher W2 abgekühlt und danach über die Pumpe P2 wieder in den Reaktor zurückgeführt.

Des Kreisgas wurde unter gleichbleibendem Druck (3 bar) mittels des Wärmetausches W3 auf 20 _{°} C abgekühlt und im Abscheider A in eine produkthaltige Flüssigphase und die Kreisgasphase getrennt, welche über den Kompressor P1 in den Reaktor zurückgeführt wurde.

Über das Entspannungsventil D4 wurden pro Stunde 2.71 kg Rohprodukt gewonnen, welches sich im wesentlichen wie folgt zusammensetzte
n-Nonanal 2.16 kg
iso-Nonanal 0.24 kg
Nonanole 0.03 kg
Octene 0.25 kg
Octan 0.03 kg
2.71 kg

Die Ausbeute an dem erwünschten Verfahrensprodukt n-Nonanal betrug somit 77 %, bezogen auf das eingesetzte Octen, und das n/iso-Verhältnis der Nonanale lag bei 9:1.

Der als Maß für die Katalysatoraktivität dienende Olefinumsatz betrug anfangs 89 % nach einer Woche Betriebszeit 88 %, nach 3 Wochen 86 % und nach 6 Wochen 85 %.

### Vergleichsbeispiel 1

### > > erkömmliche Kreisgasfahrweise bei gleicher Hydroformylierungstemperatur

Die Hydroformylierung des Octens wurde auf gleiche Weise wie im Verfahrensbeispiel vorgenommen jedoch wurde aus R lediglich die Gasphase, also das Kreisgas, entnommen, aus welchem im Abscheider A das Rohprodukt flüssig abgeschieden wurde.

Die Ergebnisse entsprechen im wesentlichen denen des Verfahrensbeispiels, jedoch waren zum gasförmigen Austrag der Verfahrensprodukte 210 Nm³/h Kreisgas erforderlich, d.h hier betrug das Kreisgas/Frischgasverhältnis 223: 1.

Dieses wirtschaftlich ungünstige Verhältnis verursachte im Vergleich zum Verfahrersbeispiel einen Mehraufwand an Energie von etwa 30 %.

### Vergleichsbeispiel 2

### » erkömmliche Kreisgasfahrweise bei höherer Hydroformylierungstemperatur

Im Unterschied zum Verfahrensbeispiel wurde die Hydroformylierung des Octens unter sonst gleichen Bedingungen bei einer Temperatur vorgenommen, bei welcher das Kreisgas/Frischgas-Verhältnis etwa das gleiche war wie beim Verfahrensbeispiel. Diese Temperatur betrug etwa 170°C.

Das Kreisgas wurde unmittelbar in den Abscheider A geführt und dort von den flüssigen Bestandteilen befreit.

Zwar entsprechen das Kreisgas/Frischgas-Verhältnis und der Gesamtenergieaufwand etwa den Werten des Verfahrensbeispiels, jedoch verschlechterte sich die Ausbeute an n-Nonanal erheblich und betrug nur noch 0.68 kg/h (= 25 %). Daneben fielen 0.29 kg/h iso-Nonanal. 0.97 kg/h Nonanole. 0.43 kg/h Octene und 0.23 kg/h Octan als unerwünschte Produkte an.

Außerdem sank der Olefinumsatz zu den Hydroformylierungsprodukten infolge der nachlassenden Katalysatoraktivität, die ihrerseits durch die höhere thermische Beanspruchung verursacht wurde, von anfangs 70 % nach 7 Tagen auf 60 %.

## Patentansprüche

1. Verfahren zur kontinuierlichen Hydroformylierung von a-Olefinen mit 5 bis 12 C-Atomen bei 2 bis 30 bar und 80 bis 130 ° C mit Hilfe von Rhodium-Komplexverbindungen als Katalysatoren, die schwer flüchtige Verbindungen der allgemeinen Formel I 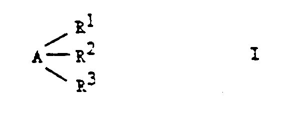 in der A für Phosphor, Arsen. Antimon oder Wismut stent und in der R¹⁻R³ organische Reste bedeuten, als Liganden enthalten, mittels Kreisgasfahrweise, dadurch gekennzeichnet, daß man das aus flüssigen und gasförmigen Bestandteilen bestehende Hydroformylierungsgemisch dem Reaktor entnimmt, in einer Entgasungskolonne kurzzeitig höheren Temperaturen und/oder niedrigeren Drücken aussetzt und dabei gleichzeitig eine Trennung des Gemisches in eine Gas- und eine Flüssigphase vornimmt, die Gasphase in einem Produktabscheider in Produkt und Kreisgas trennt und das Kreisgas sowie die Flüssigphase der Entgasungskolonne wieder in den Reaktor zurückführt.

2. Verfahren nach den Ansprüchen 1 bis 3, dadurch gekennzeichnet, daß die Entgasung in der Entgasungskolonne bei einer um 10 bis 50 °C höheren Temperatur als die Hydroformylierung vorgenommen wird.

3. Verfahren nach den Ansprüchen 1 bis 4, dadurch gekennzeichnet, daß die Entgasung in der Entgasungskolonne bei geringerem Druck als die Hydroformylierung vorgenommen wird.

## Claims

1. A process for the continuous hydroformylation of an a-olefin of 5 to 12 carbon atoms under from 2 to 30 bar and at from 80 to 130 _{°} C with the aid of, as a catalyst, a rhodium complex which contains, as ligands, sparingly volatile compounds of the formula I 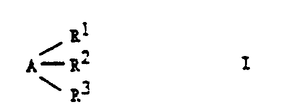 where A is phosphorus, arsenic, antimony or bismuth and R¹, R² and R³ are each organic radicals, using a recycle gas procedure, wherein the hydroformylation mixture consisting of liquid and gaseous components is removed from the reactor and is subjected to relatively high temperatures and/or relatively low pressures for a short time in a devolatilization column, the mixture at the same time being separated into a gas phase and a liquid phase, the gas phase is separated into the product and the recycle gas in a separator, and the recycle gas and the liquid phase from the devolatilization column are recycled to the reactor.

2. A process as claimed in claim 1, wherein the devolatilization in the devolatilization column is carried out at a temperature which is from 10 to 50 _{°} C higher than the hydroformylation temperature.

3. A process as claimed in claim 1 or 2, wherein the devolatilization in the devolatilization column is carried out at a lower pressure than the hydroformylation pressure.

## Revendications

1. Procédé d'hydroformylation en continu d'a-oléfines ayant de 5 à 12 atomes de carbone sous une pression de 2 à 30 bar et à une température de 80 à 130°C à l'aide de composés complexes du rhodium comme catalyseurs, qui comportent comme ligands des composés difficilement volatilisables de formule générale 1 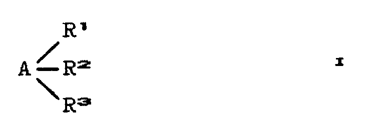 dans laquelle A représente le phosphore, l'arsenic, l'antimoine ou le bismuth et R^{I-}R³ représentent des restes organiques, avec recyclage des gaz, caractérisé en ce qu'on prélève du réacteur le mélange d'hydroformylation consistant en éléments liquides et gazeux, on le soumet pendant une courte durée à des températures supérieures et/ou à des pressions inférieures dans une colonne de dégazage, en effectuant en outre simultanément une séparation du mélange en une phase gazeuse et une phase liquide, on sépare la phase gazeuse, dans un séparateur de produits, en produit et gaz de recyclage et on recycle de nouveau au réacteur le gaz de recyclage ainsi que la phase liquide provenant de la colonne de dégazage.

2. Procédé selon la revendication 1, caractérisé en ce que le dégazage dans la colonne appropriée sera effectué à une température supérieure de 10 a 50 ° C a celle de l'hydroformylation.

3. Procédé selon l'une ou l'autre des revendications 1 et 2, caractérisé en ce que le dégazage dans la colonne appropriée sera effectué à une pression plus faible que celle de l'hydroformylation.
